# FASCICULE DE BREVET EUROPEEN

(11) **EP 1 330 279 B1**
(45) Date de publication et mention de la délivrance du brevet: **13.10.2004**
(21) Numéro de dépôt: 01980626.4
(22) Date de dépôt: 22.10.2001
(51) Int. Cl.: A61M 5/32, A61M 5/50

(54) **SERINGUE D'INJECTION A PROTECTEUR D'AIGUILLE DEPLACABLE**
INJEKTIONSSPRITZE MIT EINEM VERSCHIEBBAREN NADELSCHUTZ
INJECTION SYRINGE WITH MOBILE NEEDLE GUARD

(30) Priorité: 03.11.2000 FR 0014138
(43) Date de publication de la demande: 30.07.2003
(73) Titulaire: SEDAT, 69540 Irigny (Rhône) (FR)
(72) Inventeur: DENOLLY, Pascal, F-38200 Jardin (FR)
(74) Mandataire: Blot, Philippe Robert Emile
(86) Numéro de dépôt international: PCT/FR2001/003277
(87) Numéro de publication internationale: WO 2002/036186

(56) Documents cités:
- WO-A-00/56383
- WO-A-93/00949
- US-A- 4 887 998
- US-A- 5 882 342

## Description

La présente invention concerne une seringue d'injection du type comportant un corps de seringue muni d'une aiguille d'injection et un poussoir d'actionnement monté déplaçable dans le corps, la seringue comportant un protecteur mobile de protection de l'extrémité d'injection de l'aiguille, lequel protecteur est déplaçable par rapport au corps entre une position escamotée dans le corps en retrait de l'extrémité d'injection de l'aiguille et une position active de protection dans laquelle l'extrémité avant du protecteur se trouve en avant de l'extrémité d'injection de l'aiguille, le protecteur et le corps comportant des reliefs associés en saillie et en creux de maintien du protecteur dans une position escamotée.

Une seringue de ce type est décrite par exemple dans le document FR-A-2.757.066.

Dans ce document, le protecteur mobile de l'extrémité d'injection de l'aiguille comporte des jambages pénétrant à l'intérieur de la seringue.

Les jambages comportent des saillies adaptées pour coopérer avec la paroi avant du corps de la seringue afin de maintenir le protecteur dans sa position escamotée. L'élasticité des jambages du protecteur permet le dégagement des saillies et ainsi la libération du protecteur.

En pratique, on constate que si un appui très violent est exercé sur le poussoir d'actionnement lors de l'injection, la pression exercée sur le protecteur d'aiguille est telle que celui-ci peut être libéré et déplacé vers l'avant. Le piston intermédiaire perforable de la seringue se trouvant déplacé vers l'avant, et il n'est alors plus possible d'injecter la totalité du contenu de la seringue.

L'invention a pour but d'apporter une solution à ce problème en limitant en particulier les risques de déplacement involontaire du protecteur d'aiguille vers sa position active de protection au cours de l'injection.

A cet effet, l'invention a pour objet une seringue du type précité, caractérisée en ce qu'elle comporte un organe escamotable de retenue positive de l'engagement desdits reliefs associés en saillie et en creux, lorsque le protecteur est dans une position escamotée, et en ce que le poussoir d'actionnement et ledit organe de retenue sont adaptés pour un escamotage dudit organe de retenue par rapport au protecteur d'aiguille, sous l'action de l'enfoncement du poussoir d'actionnement dans le corps, en fin d'injection, assurant une libération de la retenue positive de l'engagement des reliefs associés en saillie et en creux.

Suivant des modes particuliers de réalisation, la seringue peut comporter l'une ou plusieurs des caractéristiques suivantes :
- le protecteur d'aiguille et l'organe escamotable de retenue sont venus de matière ;
- l'organe escamotable de retenue et le protecteur d'aiguille sont initialement solidarisés par un lien frangible ;
- lesdits reliefs associés en saillie et en creux comportent au moins un évidement ménagé dans le corps de seringue et au moins un bras élastique portant une saillie extérieure adaptée pour être reçue dans un évidement associé du corps, et ledit organe de retenue comporte au moins une butée de maintien de la ou chaque saillie extérieure dans l'évidement associé, avant escamotage dudit organe de retenue ;
- lesdits bras élastiques sont ménagés par le tronçon d'extrémité arrière du protecteur d'aiguille tourné vers le piston arrière d'actionnement ;
- ladite aiguille d'injection se prolonge axialement à l'intérieur du corps jusqu'à une extrémité arrière à l'écart de laquelle est initialement disposée, dans le corps, une paroi transversale perforable, l'aiguille et ladite paroi transversale étant déplaçables l'une par rapport à l'autre depuis une position initiale dans laquelle la paroi transversale est écartée de l'aiguille jusqu'à une position finale dans laquelle la paroi transversale est transpercée par ladite aiguille ;
- ledit organe de retenue comporte un passage de guidage axial de l'extrémité arrière de l'aiguille, lors de la perforation de la paroi transversale ;
- ladite paroi transversale est déformable axialement sous l'action du poussoir d'actionnement agissant sur ledit organe de retenue ;
- ledit protecteur d'aiguille et ladite paroi transversale sont initialement espacés l'un de l'autre, et ils comportent des profils complémentaires de solidarisation axiale par enclenchement élastique ;
- le protecteur d'aiguille et la paroi transversale comportent des moyens de solidarisation axiale au moins lorsque l'aiguille et la paroi transversale sont dans leur position finale, et le protecteur d'aiguille et le corps de seringue comportent des profils complémentaires en saillie et en creux interdisant le déplacement du protecteur d'aiguille vers le poussoir d'actionnement lorsque l'aiguille et la paroi transversale sont dans leur position finale ;
- le protecteur d'aiguille comporte au moins deux jambes s'étendant généralement parallèlement, lesquelles jambes sont venues de matière et reliées l'une à l'autre à leur extrémité arrière tournée vers le poussoir d'actionnement ;
- le protecteur d'aiguille comporte une bague rapportée reliant les extrémités des jambes opposées à leur extrémité tournée vers le piston d'actionnement ;
- le protecteur d'aiguille et le corps de seringue comportent des profils complémentaires en saillie et en creux interdisant le déplacement du protecteur d'aiguille vers le poussoir d'actionnemeht lorsque le protecteur d'aiguille est dans sa position escamotée initiale ; et
- le corps de seringue comporte au moins une lame élastique et le protecteur d'aiguille comporte au moins une encoche adaptée pour coopérer avec ladite lame élastique afin d'interdire le déplacement du protecteur d'aiguille vers le poussoir d'actionnement lorsque le protecteur d'aiguille est dans sa position active de protection.

L'invention sera mieux comprise à la lecture de la description qui va suivre, donnée uniquement à titre d'exemple et faite en se référant aux dessins sur lesquels :
- la figure 1A est une vue en coupe longitudinale d'une seringue selon l'invention, avant utilisation;
- la figure 1B est une vue en coupe longitudinale de la seringue de la figure 1A dans un plan de coupe décalé angulairement de 90° ;
- la figure 1C est une vue à plus grande échelle de l'extrémité arrière du protecteur d'aiguille dans le plan de coupe de la figure 1B ;
- la figure 1D est une vue à plus grande échelle de l'extrémité avant du protecteur d'aiguille dans le plan de coupe de la figure 1B ;
- la figure 2 est une vue en perspective et en coupe longitudinale du support d'aiguille de la seringue selon l'invention ;
- la figure 3A est une vue en perspective du protecteur d'aiguille de la seringue selon l'invention avant montage dans la seringue ;
- la figure 3B est une vue en perspective et en coupe longitudinale du protecteur d'aiguille avant montage dans la seringue ;
- les figures 4A, 48, 4C et 4D sont des vues analogues aux figures 1A, 1B, 1C et 1D, respectivement, après perforation du piston intermédiaire ;
- les figures 5A, 5B, 5C et 5D sont des vues analogues aux figures 1A, 1B, 1C et 1D, respectivement, en fin d'injection, avant libération du protecteur d'aiguille ;
- les figures 6A, 6B, 6C et 6D sont des vues analogues aux figures 1A, 1B, 1C et 1D, respectivement, après libération du protecteur d'aiguille ;
- les figures 7A, 7B, 7C et 7D sont des vues analogues aux figures 1A, 1B, 1C et 1D, respectivement, lors de la mise en place du protecteur d'aiguille;
- les figures 8A, 8B, 8C et 8D sont des vues analogues aux figures 1A, 1B, 1C et 1D, respectivement, à l'issue de la mise en place du protecteur d'aiguille dans sa position active de protection ;
- la figure 9 est une vue en coupe longitudinale de l'extrémité avant d'un autre mode de réalisation d'un protecteur d'aiguille utilisé dans une seringue selon l'invention ;
- les figures 10A et 10B sont des vues analogues aux figures 1A et 1B d'une variante de réalisation d'une seringue selon l'invention ;
- les figures 11A et 11B sont des vues respectivement en coupe longitudinale et en extérieur du poussoir d'actionnement de la seringue des figures 10A et 10B, celui-ci étant représenté respectivement dans son état rétracté et dans son état déployé ;
- la figure 11C est une vue en section prise suivant la ligne XI-XI du poussoir d'actionnement de la figure 11A ;
- les figures 12A, 12B et 12C sont des vues analogues aux figures 5A, 5B et 5C de la variante de réalisation de la seringue ;
- les figures 13A, 13B, 13C sont des vues analogues aux figures 6A, 6B et 6C de la variante de réalisation de la seringue ; et
- les figures 14A, 14B et 14C sont des vues analogues aux figures 8A, 8B et 8C de la variante de réalisation de la seringue.

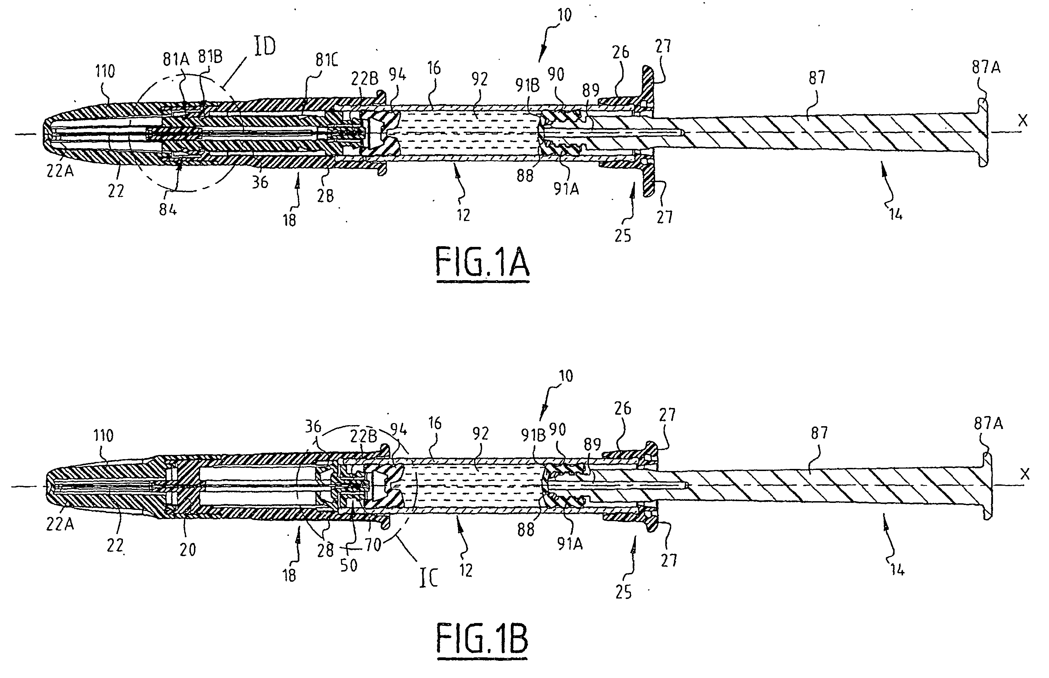

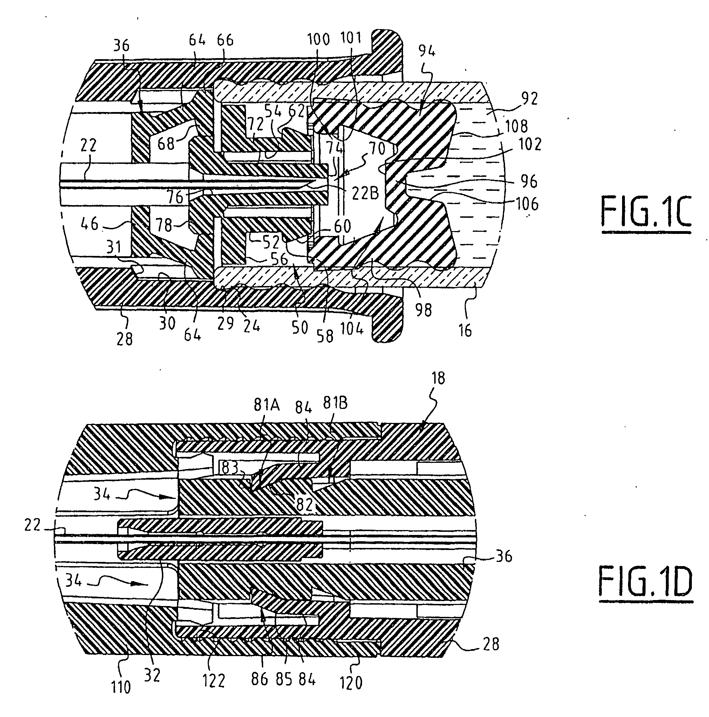

La seringue d'injection 10 représentée sur la figure 1, de forme générale de révolution d'axe X-X, est une seringue à usage unique. Elle est proposée prête à l'emploi et contenant déjà un fluide médical à injecter. Elle comporte essentiellement un corps de seringue 12 allongé et un poussoir d'actionnement 14 monté déplaçable à l'intérieur du corps 12.

Le corps de seringue 12 est formé d'un tube 16 à l'extrémité avant duquel est fixé un support d'aiguille 18. Ce support d'aiguille comporte une traverse 20 formant la paroi avant du corps de seringue. Cette traverse 20, visible sur la figure 1B, est munie d'une aiguille d'injection traversante 22. Celle-ci comporte une extrémité avant d'injection 22A faisant saillie par rapport au corps 12. L'aiguille se prolonge axialement à l'intérieur du corps 12 jusqu'à une extrémité arrière 22B.

Le tube 16 est réalisé par exemple en verre et a une section circulaire. Son extrémité avant est munie extérieurement d'un bourrelet périphérique 24 visible sur la figure 1C. Ce bourrelet est destiné au maintien du support d'aiguille 18. A l'extrémité arrière du tube 16 est rapporté un organe de préhension 25 facilitant la préhension du corps de seringue entre l'index et le majeur. Celui-ci comporte un manchon 26 encliqueté extérieurement à l'extrémité du tube 16 et deux pattes 27 diamétralement opposées pour l'appui des doigts.

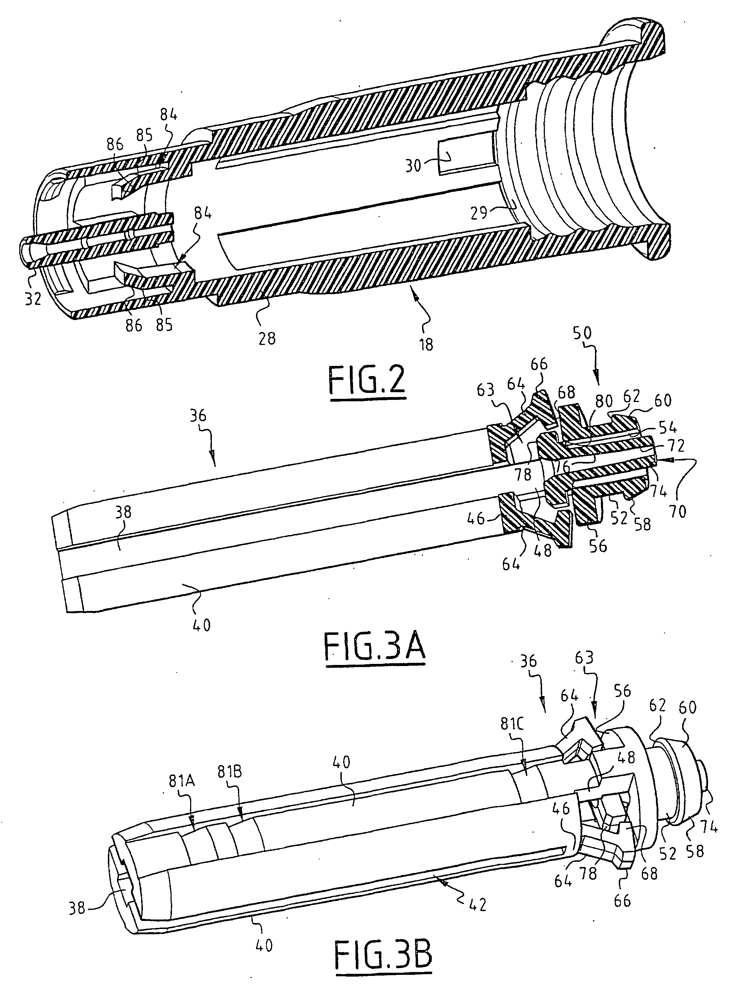

Le support d'aiguille 18 est représenté en coupe sur la figure 2. Il est délimité extérieurement par un manchon 28. La traverse 20 est venue de matière avec le manchon 28 au voisinage de son extrémité avant où elle s'étend suivant un diamètre du manchon. Sur la paroi intérieure du manchon 28 est ménagée, au voisinage de son extrémité ouverte opposée à la traverse 20, une gorgé périphérique 29 de réception du bourrelet 24.

Immédiatement en avant de la gorge périphérique 29, du côté de la traverse 20, deux évidements 30 sont ménagés dans la surface intérieure du manchon 28. Ces évidements 30 présentent, à leur extrémité tournée vers la traverse 20, une rampe 31 assurant une réduction progressive de la profondeur de l'évidement en direction de la traverse 20, c'est-à-dire vers l'extrémité avant de la seringue.

La traverse 20 présente un plot axial 32 venu de matière qui assure la retenue de l'aiguille d'injection 22. Ce plot, visible sur la figure 1D, est dirigé vers l'extrémité d'injection 22A de l'aiguille et est reçu à l'intérieur de l'espace délimité par le manchon 28.

Deux passages 34 identiques, en forme de demi-lune, sont délimités de part et d'autre de la cloison 20 à l'intérieur du manchon 28.

Ces passages 34 assurent le guidage d'un protecteur d'aiguille 36 représenté seul sur les figures 3A et 3B.

Le protecteur d'aiguille 36 présente une forme généralement allongée. Il est traversé axialement de part en part par un conduit axial 38. Le protecteur 36 est fendu longitudinalement sur l'essentiel de sa longueur depuis son extrémité avant et délimite ainsi deux jambes 40 s'étendant parallèlement l'une à l'autre de part et d'autre de la fente notée 42. Les deux jambes sont reliées l'une à l'autre au voisinage de l'extrémité arrière du protecteur d'aiguille par une couronne 46 venue de matière. Les deux jambes 40 et la couronne 46 forment le corps du protecteur. Chacune des jambes est prolongée par deux piliers 48 s'étendant au-delà de la couronne 46. Les piliers 48 sont reliés à l'opposé des jambes 40 par une tête 50 de révolution s'étendant suivant l'axe du protecteur d'aiguille. Cette tête 50 présente une paroi généralement cylindrique 52 délimitant un passage interne 54. A son extrémité tournée vers les jambes 40, la paroi cylindrique 52 présente une collerette extérieure 56. A son autre extrémité, la paroi cylindrique 52 est bordée par un bourrelet d'encliquetage extérieur 58 présentant une surface tronconique tournée vers l'extrémité de la tête 50, cette surface tronconique s'achevant par un épaulement 62 faisant face à la collerette 56.

Dans l'intervalle 63 délimité entre la couronne 46 reliant les jambes et la tête 50 s'étendent deux bras élastiques 64 reliés à l'une de leurs extrémités à la couronne 46 avec laquelle ils sont venus de matière. Ces bras sont généralement rectilignes et présentent une extrémité libre. Au repos, ils s'étendent dans un plan passant par l'axe du protecteur d'aiguille et s'écartent progressivement de l'axe de celui vers leur extrémité libre.

A son extrémité libre, chaque bras présente une saillie extérieure 66 tournée à l'opposé de l'axe du protecteur. Cette .saillie est adaptée pour s'engager dans un évidemment 30 du support d'aiguille.

En outre, à cette même extrémité, chaque bras 64 présente une saillie intérieure 68 tournée vers l'axe du protecteur.

Un organe escamotable 70 de retenue positive des bras 64 dans une position écartée est initialement disposé partiellement entre les extrémités des bras 64. Cet organe de retenue comporte un corps tubulaire 72 s'étendant initialement à l'intérieur du conduit 54 suivant l'axe du protecteur. La longueur du corps tubulaire 72 est telle que son extrémité arrière notée 74 fait saillie au-delà de l'extrémité arrière de la tête 50 suivant l'axe du protecteur.

Intérieurement, le corps tubulaire 72 définit un passage généralement tronconique 76 de guidage et de centrage de l'extrémité arrière 22B de l'aiguille.

A son extrémité avant, s'étendant initialement entre les extrémités libres des bras 64, l'organe de retenue 70 présente extérieurement deux excroissances 78 diamétralement opposées. Celles-ci sont adaptées pour former des butées d'appui pour les saillies intérieures 68 retenant ainsi les bras 64 écartés et les saillies extérieures 66 dans les évidements 30 du support d'aiguille.

A sa périphérie extérieure, l'organe de retenue 70 est relié à la tête 50 du protecteur d'aiguille par un lien frangible 80 formé d'un voile reliant l'organe de retenue et la tête 50, la tête et l'organe de retenue étant venus de matière l'un avec l'autre au travers de ce voile à l'extrémité du passage 54.

Comme représenté sur la figure 1C, avant utilisation de la seringue, l'extrémité arrière 22B de l'aiguille s'étend dans le passage 76, de sorte que l'extrémité de l'aiguille est complètement recouverte par l'organe de retenue 70.

Chaque jambe 40 du protecteur comporte, sur sa surface latérale, trois encoches 81A, 81B, 81C disposées longitudinalement dans cet ordre, depuis l'extrémité avant des jambes du protecteur jusqu'à leur extrémité arrière où les deux jambes sont liées par la couronne 46.

Les encoches 81A et 81B sont disposées à proximité l'une de l'autre au voisinage immédiat de l'extrémité avant des jambes 40. En revanche, l'encoche 81 C de chaque jambe est disposée immédiatement en avant de la couronne 46.

Les trois encoches présentent des profils identiques. Comme illustré par exemple sur la figure 1D, chaque encoche présente une rampe inclinée 82 depuis la surface latérale de la jambe vers le fond de l'encoche, suivant un sens allant de l'arrière de la jambe vers l'avant de celle-ci. Cette rampe 82 est suivie, vers l'avant de chaque jambe, par un épaulement 83 formant une butée.

Les encoches 81A, 81B et 81C constituent des reliefs en creux adaptés pour coopérer avec des reliefs en saillie associés ménagés sur le support d'aiguille 28 afin d'interdire un retour en arrière du protecteur d'aiguille depuis trois positions correspondant à chacune des encoches.

Les profils en saillie complémentaires portés par le protecteur d'aiguille 28 sont formés par des lames élastiques 84 venues de matière avec le manchon 28 du support d'aiguille. Ces lames élastiques font saillie par rapport à la surface latérale intérieure du manchon.

Les lames élastiques 84 sont liées au manchon 28 seulement à leur extrémité arrière. Elles s'étendent généralement parallèlement à l'axe de la seringue et leur extrémité libre est tournée vers l'extrémité d'injection 22A de l'aiguille. Chaque lame élastique présente un coude 85 prolongé par un tronçon saillant de blocage 86 tourné vers l'axe de la seringue. Ce tronçon de blocage saillant 86 est adapté pour être reçu successivement dans chacune des encoches 81A, 81B. En particulier, l'extrémité du tronçon de blocage 86 est adaptée pour venir en butée contre l'épaulement 83 de chacune de ces encoches et pour glisser sur les rampes 82 afin de provoquer une déformation élastique de la lame associée.

Le poussoir d'actionnement 14 comporte une tige de piston 87 ayant une section en croix et présentant, à son extrémité arrière, une pastille 87A d'appui du pouce de l'opérateur. A son extrémité opposée la tige de piston 87 présente une protubérance axiale 88 généralement tronconique et dont le diamètre décroît vers son extrémité libre. Le diamètre maximal de cette protubérance est inférieur à la section du passage 54 ménagé dans la tête du protecteur. Un logement 89 ouvert sur l'avant est ménagé axialement dans la tige de piston 87. Ce logement sert à la réception de l'extrémité arrière 22B de l'aiguille en fin d'injection. Le logement 89 présente une forme allongée suivant l'axe X-X et a une section circulaire.

Le logement 89 est obturé par un piston d'extrémité 90 en forme de cuvette formant un piston arrière de la seringue. Ce piston 90 présente une paroi latérale cylindrique 91A obturée par un disque 91B formant le fond de la cuvette. La paroi latérale 91A est munie intérieurement de bourrelets successifs. A sa surface extérieure, la tige de piston 87 présente des gorges annulaires dans lesquelles sont reçus les bourrelets successifs, assurant ainsi une solidarisation axiale du piston arrière 90 et de la tige de piston 87. Le piston arrière 90 est déformable élastiquement, notamment dans sa partie constituée du disque 91B. Le piston arrière 90 constitue une paroi transversale adaptée pour coulisser de manière étanche à l'intérieur du tube 16. Le disque 91A est perforable dans sa partie centrale.

Tel que représenté sur la figure 1A, le fluide à injecter 92 est disposé à l'intérieur du tube 16 dans un espace délimité par le piston arrière 90 et un piston intermédiaire 94.

Le piston intermédiaire 94 est représenté à plus grande échelle sur la figure 1C. Il est réalisé dans un matériau élastique tel que du caoutchouc. Il comporte une paroi transversale 96 perforable axialement dans sa région centrale. Cette paroi transversale est entourée par un manchon latéral 98 venu de matière avec celle-ci. Ce manchon latéral est adapté pour s'appliquer de manière étanche contre la surface intérieure du tube 16. A son extrémité tournée vers la tête 50 du protecteur d'aiguille, la paroi latérale 98 et la paroi transversale 96 délimitent, dans le piston intermédiaire 94, une chambre 100 ayant une forme complémentaire au profilé extérieur de la tête 50 délimitée par la surface latérale 52 et le bourrelet d'encliquetage 58. La chambre 100 s'ouvre vers le protecteur d'aiguille. Elle présente, à la périphérie de son ouverture, un rebord 101 faisant saillie radialement, adapté pour assurer la retenue ultérieure de la tête 50 dans la chambre.

Ainsi, la chambre 100 est adaptée pour recevoir l'extrémité de la tête 50 et retenir celle-ci axialement par enclenchement élastique.

Au fond de la chambre 100, la paroi transversale 96 présente une surface plane 102 tournée vers l'organe 70 de retenue. Cette surface 102 est adaptée pour s'appuyer suivant la tranche du corps de l'organe de retenue 70 depuis son extrémité 74 faisant saillie par rapport à la tête 50 du protecteur d'aiguille.

La surface 102 est formée au sommet d'une excroissance 104 ménagée au fond de la chambre 100, cette excroissance étant entourée d'un canal annulaire. L'aire de la surface plane 102 est inférieure à la section du conduit 54 au travers duquel est monté l'organe 70 de retenue.

Suivant sa face tournée vers le poussoir d'actionnement 14, le piston intermédiaire 94 présente, en son centre, un puits 106, dont la périphérie est reliée à la paroi latérale 98 par une surface tronconique 108 s'évasant progressivement en direction du poussoir d'actionnement 14.

Cette surface tronconique 108 constitue un siège d'appui pour l'extrémité du poussoir d'actionnement 14.

Comme illustré sur la figure 1C, le piston intermédiaire 94 est disposé initialement immédiatement en arrière de l'extrémité arrière du protecteur d'aiguille 36. En particulier, la tête 50 du protecteur est en dehors de la chambre 100. Dans cette position initiale, l'extrémité arrière 22B de l'aiguille est à l'écart de la paroi transversale perforable 96.

En variante, le piston intermédiaire 94 est initialement engagé autour de la tête 50 du protecteur. L'extrémité arrière 22B de l'aiguille est toutefois à l'écart de la paroi perforable 96, cette extrémité étant disposée dans le passage 76.

Par ailleurs, un capuchon 110 de protection de l'aiguille 22 est engagé autour du manchon 28 du support d'aiguille et recouvre l'extrémité d'injection 22A de l'aiguille.

Comme illustré sur la figure 1D, le capuchon 110 présente à son extrémité ouverte une jupe 120 délimitant un espace annulaire à l'intérieur duquel est reçue l'extrémité du support d'aiguille 18.

La surface latérale externe du support d'aiguille 18 et la surface latérale interne de la jupe 120 s'appliquent l'une contre l'autre.

L'une de ces surfaces présente, avantageusement, un canal hélicoïdat 122 débouchant, à une extrémité, à l'extérieur de la seringue, lorsque le capuchon est mis en place sur le support d'aiguille 18, l'autre extrémité du canal 122 débouchant à l'intérieur du capuchon.

Ce canal hélicoïdal 122 est réalisé dans la jupe 120 dans le mode de réalisation envisagé. Il assure la circulation des gaz entre l'extérieur et l'intérieur du protecteur d'aiguille lorsque celui-ci est en place sur le support d'aiguille. Ainsi, ce canal permet la stérilisation de la seringue équipée de son protecteur d'aiguille dans un autoclave ou tout autre dispositif de stérilisation.

Le montage de la seringue s'effectue de la manière suivante.

L'aiguille 22 est collée au travers du support d'aiguille 18. Le protecteur 36 est introduit dans le support d'aiguille 18 au travers de son extrémité arrière, c'est-à-dire du côté de l'extrémité 22B de l'aiguille. Le protecteur 36 est monté au travers du support d'aiguille 18 par engagement des jambes 40 dans les passages 34 entourant le plot 32. Les jambes s'étendent alors le long de la partie arrière de l'aiguille 22. Dans cette position, les extrémités en saillie 86 des lames élastiques 84 sont reçues dans les encoches 81A, interdisant un retrait du protecteur par traction depuis son extrémité arrière. Le capuchon avant 110 est alors mis en place par emmanchement de celui-ci autour du manchon 28.

Le support d'aiguille 18, ainsi muni du capuchon 110 et du protecteur d'aiguille 36 peut être manipulé sans risque de dégradations des extrémités de l'aiguille, celle-ci étant protégée à ses deux extrémités. En particulier, il peut être distribué sur les chaînes de fabrication dans des bols vibrants.

Parallèlement à l'assemblage du support d'aiguille, le tube 16 est muni de l'organe de préhension 25. Il est rempli du fluide 92 disposé entre le piston intermédiaire 94 et le poussoir d'actionnement 14. Le support d'aiguille 18 est mis en place par encliquetage à l'extrémité avant du tube 16, comme représenté sur la figure 1C.

Afin de procéder à l'injection, l'opérateur retire le capuchon 110. De manière classique, l'opérateur exerce alors avec le pouce une poussée sur le poussoir d'actionnement 14 en prenant appui sous les pattes 27 avec l'index et le majeur.

La pression ainsi exercée, transmise par l'intermédiaire du liquide 92 au piston intermédiaire 94, provoque le déplacement de celui-ci vers l'extrémité arrière 22B de l'aiguille.

Le piston 94 s'engage alors sur la tête 50 du protecteur d'aiguille et s'y trouve maintenu par enclenchement élastique du rebord rentrant 101 bordant l'ouverture de la chambre en arrière du bourrelet d'encliquetage 58 de la tête.

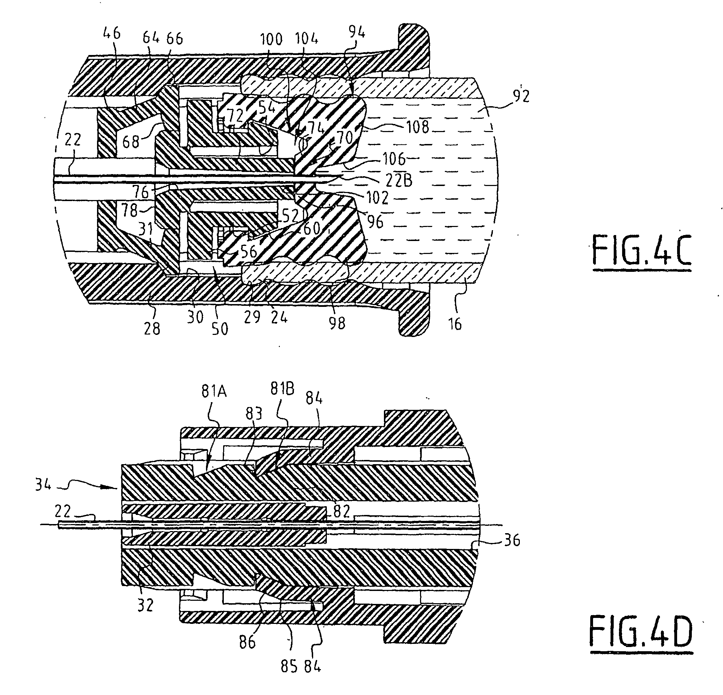

Dans cette position, la surface plane 102 ménagée au fond de la chambre s'appuie sur l'extrémité arrière de l'organe escamotable 70 de retenue, comme illustré sur la figure 4C.

Le déplacement ultérieur du piston 94 associé au protecteur d'aiguille conduit à ce que le piston 94 s'empale sur l'extrémité arrière 22B de l'aiguille. En particulier, la paroi 96 du piston intermédiaire se trouve perforée au cours de son mouvement.

Lors de la perforation de la paroi transversale 96 par l'extrémité arrière 22B de l'aiguille, le conduit 76 traversant de part en part l'organe de retenue 70 assure un maintien de l'aiguille exactement suivant l'axe de la seringue, garantissant ainsi que la perforation de la paroi s'effectue en son milieu et exactement suivant la direction de l'axe de la seringue.

Un déplacement ultérieur aisé du piston 94 sur tout le tronçon intérieur de l'aiguille 22 est ainsi garanti.

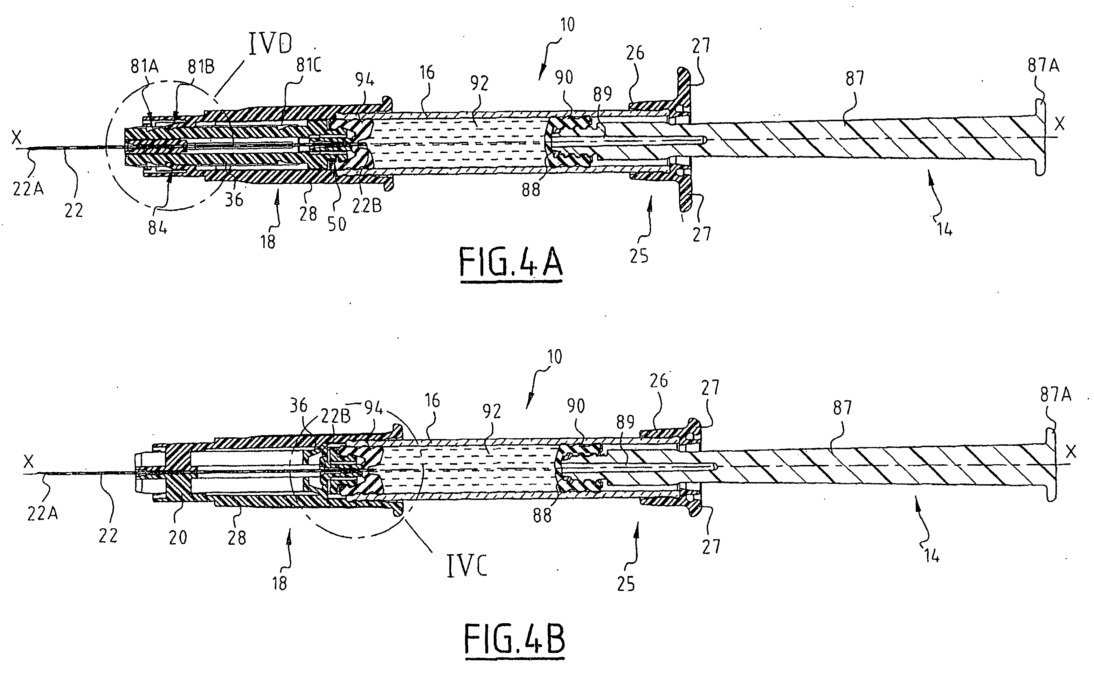

La poursuite de l'enfoncement du poussoir d'actionnement 14 provoque le déplacement vers l'avant du protecteur d'aiguille 36 accouplé au piston intermédiaire 94. Ce déplacement s'effectue jusqu'à ce que les saillies extérieures 66 s'appuient contre la rampe 31 formée à l'avant de chaque évidement 30. Dans cette position illustrée aux figures 4A, 4B et 4C, le déplacement du protecteur d'aiguille est interrompu, par la butée des saillies extérieures 66 contre la rampe 31. En effet, du fait de la présence des excroissances 78 interposées entre les extrémités libres des bras 64, ceux-ci ne peuvent se déformer élastiquement et se rapprocher, de sorte que les saillies extérieures 66 sont emprisonnées dans les évidements 30, interdisant ainsi un déplacement axial ultérieur du protecteur d'aiguille 36.

Dans cette position, et comme illustré sur la figure 4D, les tronçons en saillie 86 des lames élastiques 84 sont reçues dans les entailles 81B, interdisant un déplacement vers l'arrière du protecteur d'aiguille.

Le protecteur d'aiguille est alors immobilisé axialement suivant les deux sens. Cette immobilisation est très fiable puisque, d'une part, la forme particulière des lames 84 et des encoches 81B interdit un retour en arrière du protecteur et, d'autre part, les excroissances 78 assurent une retenue positive des saillies 66 dans les évidements 30.

Après une purge de la seringue, l'extrémité d'injection 22A de l'aiguille est introduite dans les tissus du patient.

Après introduction de l'aiguille, il est possible de procéder à un "test veine", c'est-à-dire à une aspiration d'une goutte de sang au travers de l'aiguille par traction sur le poussoir d'actionnement 14. Un tel test ne crée aucun risque de mouvement du piston intermédiaire 94, celui-ci étant retenu par le protecteur d'aiguille 36 qui est lui-même immobilisé par les lames élastiques 84 engagées dans les encoches 81B.

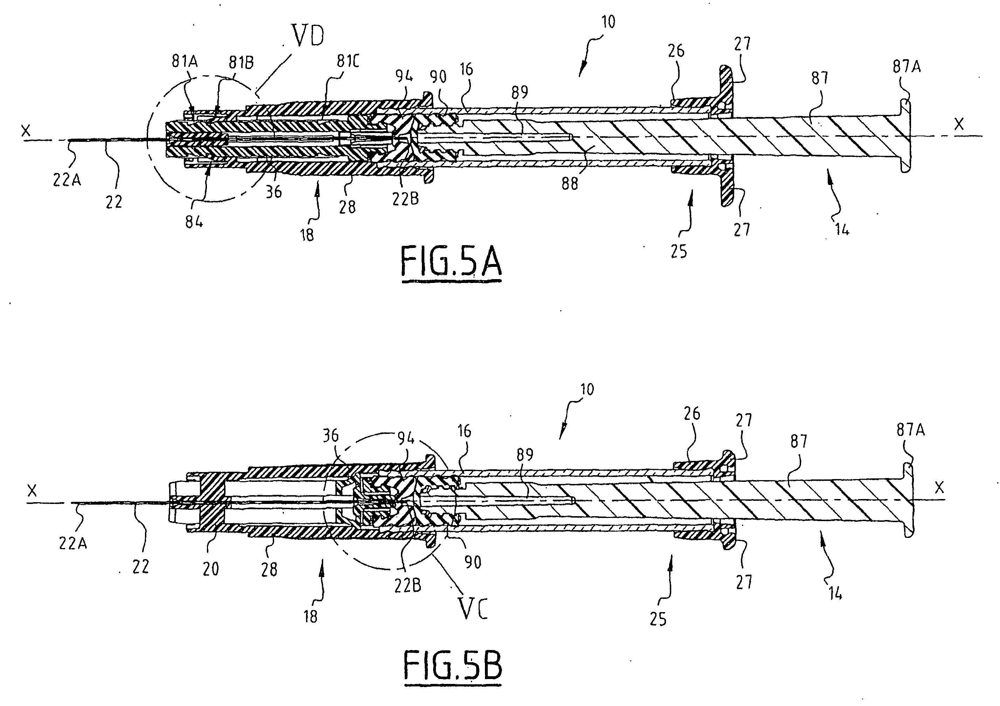

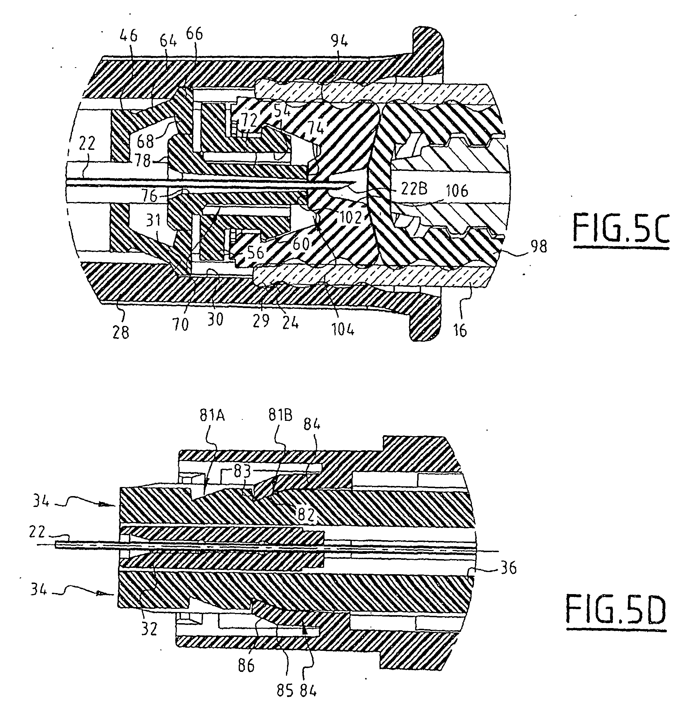

Après achèvement du test, le fluide 92 est alors injecté au travers de l'aiguille 22 sous l'action de la poussée du poussoir d'actionnement 14 enfoncé jusqu'à la position représentée sur les figures 5A, 5B, 5C et 5D. Dans cette position, l'essentiel du fluide 92 est injecté et le piston arrière 90 vient au contact de la surface arrière du piston intermédiaire 94.

Pendant toute l'injection, le protecteur d'aiguille 36 est maintenu immobile par l'engagement des tronçons en saillie 86 des lames élastiques dans les encoches 81B et par le maintien des saillies extérieures 66 dans les évidements 30; ce maintien étant garanti par les excroissances 84 de l'organe escamotable de retenue 70.

Le maintien du protecteur est réalisé de manière fiable quel que soit l'effort appliqué sur le poussoir 14 du fait du blocage positif du protecteur assuré par l'organe de retenue 70.

A l'issue de la phase d'injection, le piston arrière 90 vient en butée contre le piston intermédiaire 94, lequel est porté par le protecteur d'aiguille qui est immobilisé axialement par appui des saillies externes 86 contre les rampes 31.

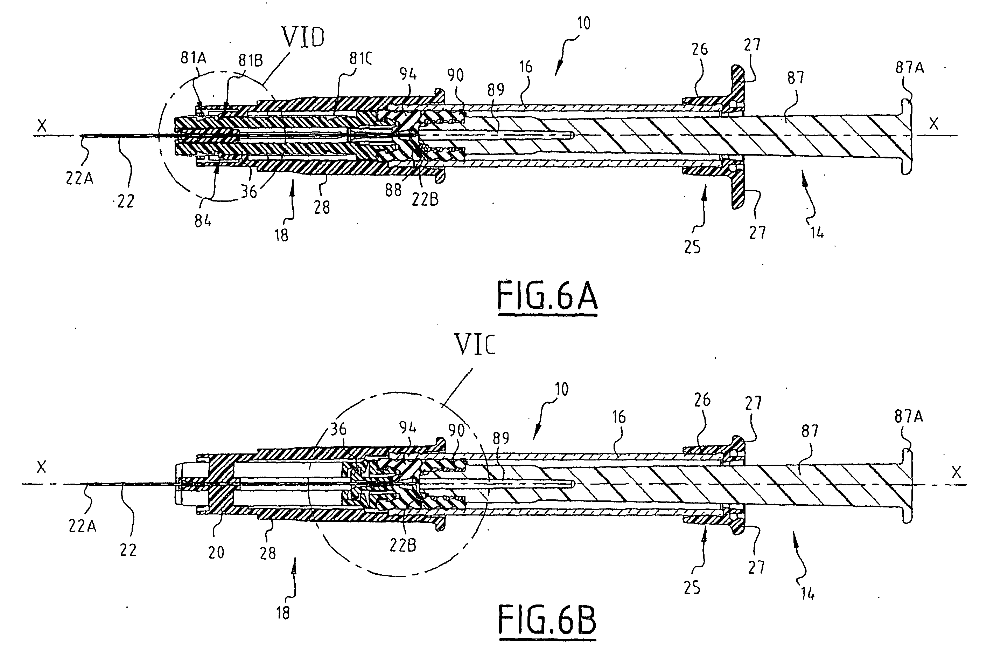

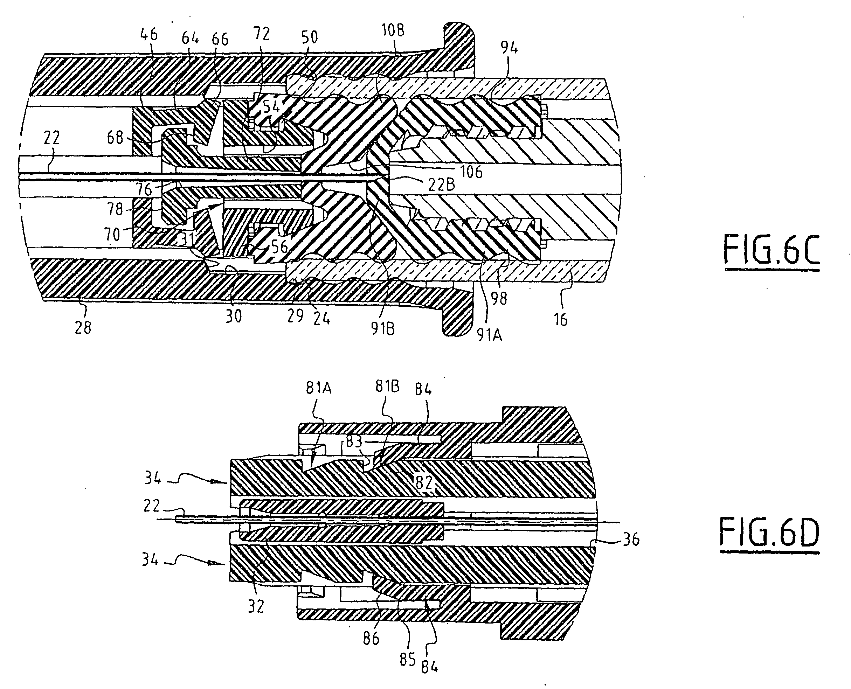

Lors de la poursuite de l'enfoncement du poussoir d'actionnement 14, et comme illustré sur les figures 6A, 68, 6C et 6D, l'extrémité tronconique 88 de la tige de piston provoque une déformation élastique du disque transversal 91B du piston arrière 90. Celui-ci s'appuie sur le siège 108 formé sur le piston intermédiaire 94. Sous l'action de la poussée exercée par la tige de piston 87 au travers du piston arrière 90, le piston intermédiaire 94 se déforme en se comprimant axialement. Cette déformation provoque ainsi un déplacement vers l'avant de la paroi transversale 96 et en particulier de la surface 102 en appui sur l'extrémité 74 de l'organe escamotable de retenue 70. Sous l'action de cette poussée, l'organe de retenue 70 se détache de la fête 50 du protecteur d'aiguille, la liaison frangible 80 se trouvant rompue sous l'effet de la force de poussée.

Dans ces conditions, l'organe de retenue 70 ainsi désolidarisé du reste du protecteur d'aiguille 36 se déplace axialement vers une position escamotée en direction de l'extrémité avant 22A de l'aiguille. En particulier, les excroissances 78 formant initialement une cale entre les saillies internes 68 se trouvent déplacées en avant de ces saillies, libérant ainsi le mouvement des extrémités libres des bras 64.

Comme illustré sur la figure 6C, lors du déplacement de la tige de piston 87, les bras 64 libérés de l'organe de retenue 70 se trouvent déformés élastiquement sous l'action de la coopération des saillies externes 66 avec les rampes 31 qui forment des surfaces de came.

Le protecteur d'aiguille 36 est alors libre de se déplacer axialement vers l'extrémité d'injection de l'aiguille sous l'action de la poussée exercée par le poussoir d'actionnement 14.

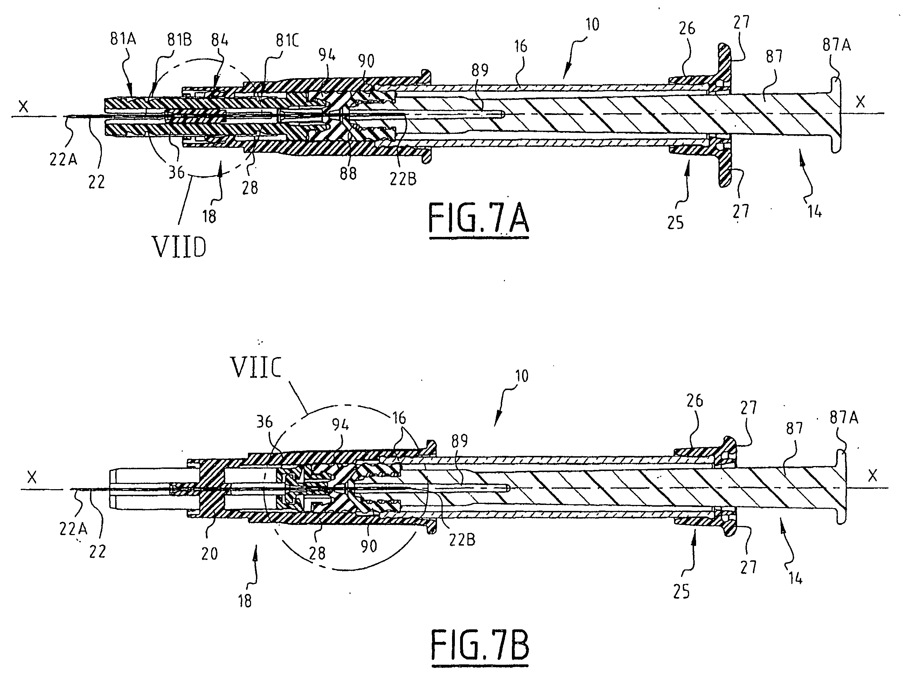

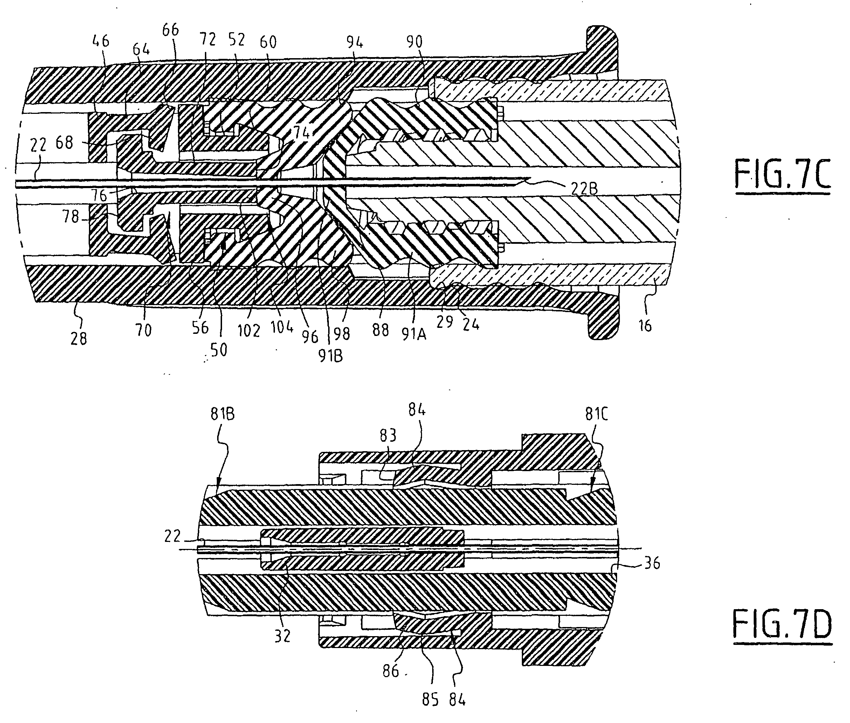

Lors dé la poursuite de l'enfoncement du poussoir 14, et comme illustré sur les figures 7A, 7B, 7C et 7D, les jambes 40 du protecteur d'aiguille se déplacent progressivement le long du tronçon de l'aiguille faisant saillie hors du support d'aiguille 18.

Lors du déplacement du protecteur d'aiguille 36, l'extrémité arrière 22B de l'aiguille pénètre à l'intérieur de la chambre 89 après avoir perforé le piston arrière 90.

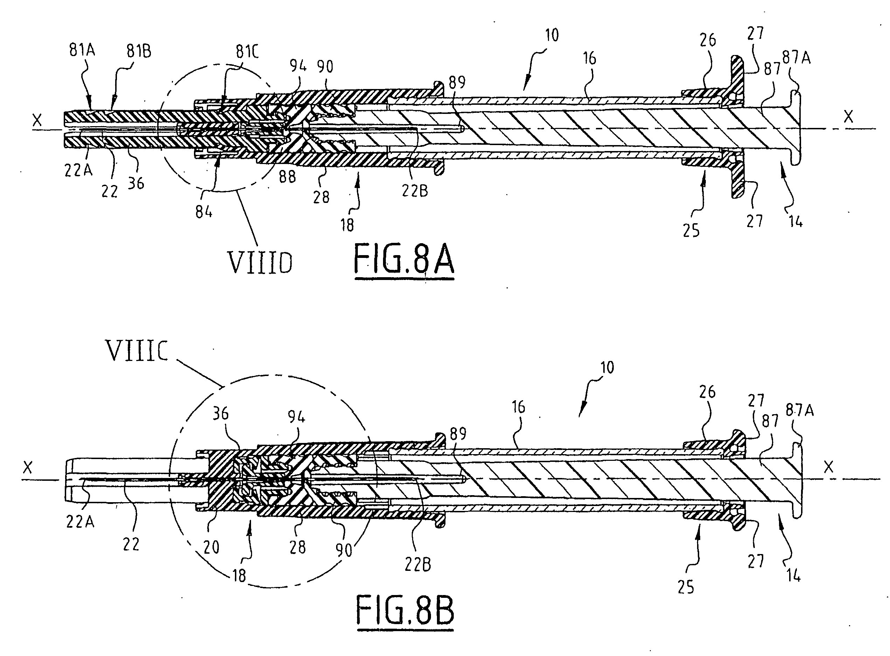

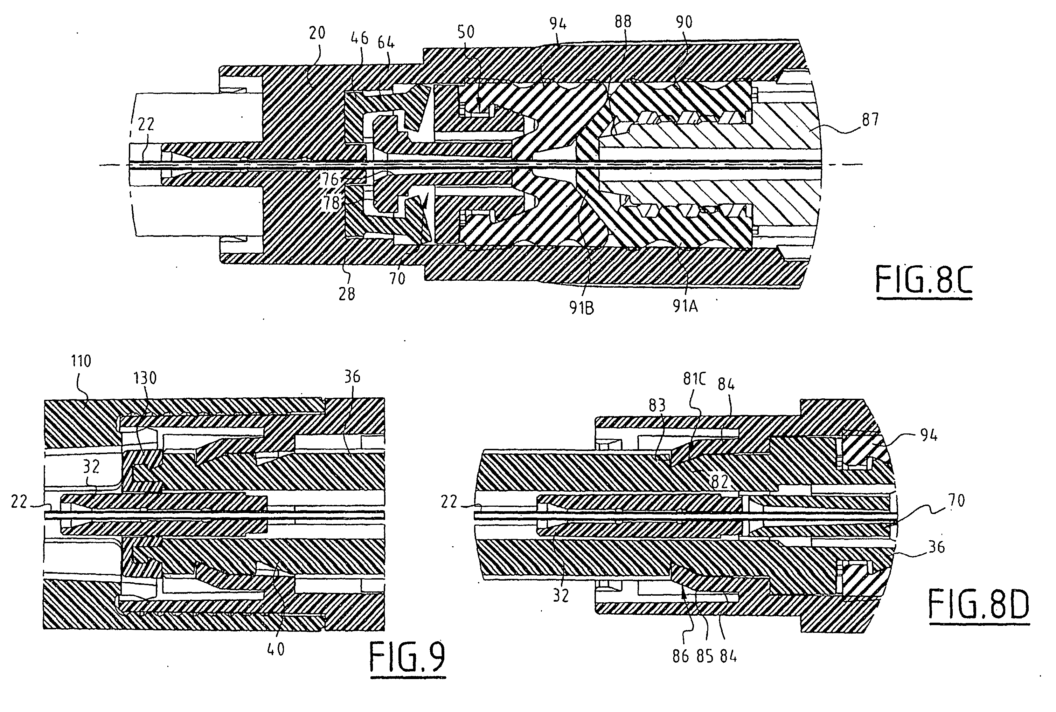

Le déplacement du protecteur d'aiguille 36 s'effectue sous l'action du poussoir 14 jusqu'à ce que le protecteur d'aiguille entre en contact avec la traverse 20 comme illustré sur les figures 8A, 8B et 8C. Dans cette position, et comme illustré sur la figure 8D, les extrémités des lames élastiques 84 sont reçues dans les encoches 81C, assurant ainsi un blocage axial du protecteur d'aiguille, évitant son retour en arrière.

Lorsque le protecteur d'aiguille est dans sa position active de protection, comme illustré sur les figures 8A et 8B, l'extrémité avant du protecteur d'aiguille s'étend en avant de l'extrémité avant 22A de l'aiguille d'injection, évitant tout risque de piqûre depuis l'extrémité de l'aiguille.

Sur la figure 9 est représentée une variante de réalisation d'un protecteur d'aiguille mis en oeuvre dans une seringue telle que décrite aux figures précédentes.

Dans ce mode de réalisation, les extrémités libres des jambes 80 sont équipées d'une bague 130 rapportée par encliquetage ou tout autre moyen approprié de solidarisation. Cette bague assure un cerclage et une solidarisation des extrémités libres des jambes. Elle évite tout risque de voir les jambes du protecteur d'aiguille écartées alors que celui-ci est dans sa position active de protection de l'aiguille.

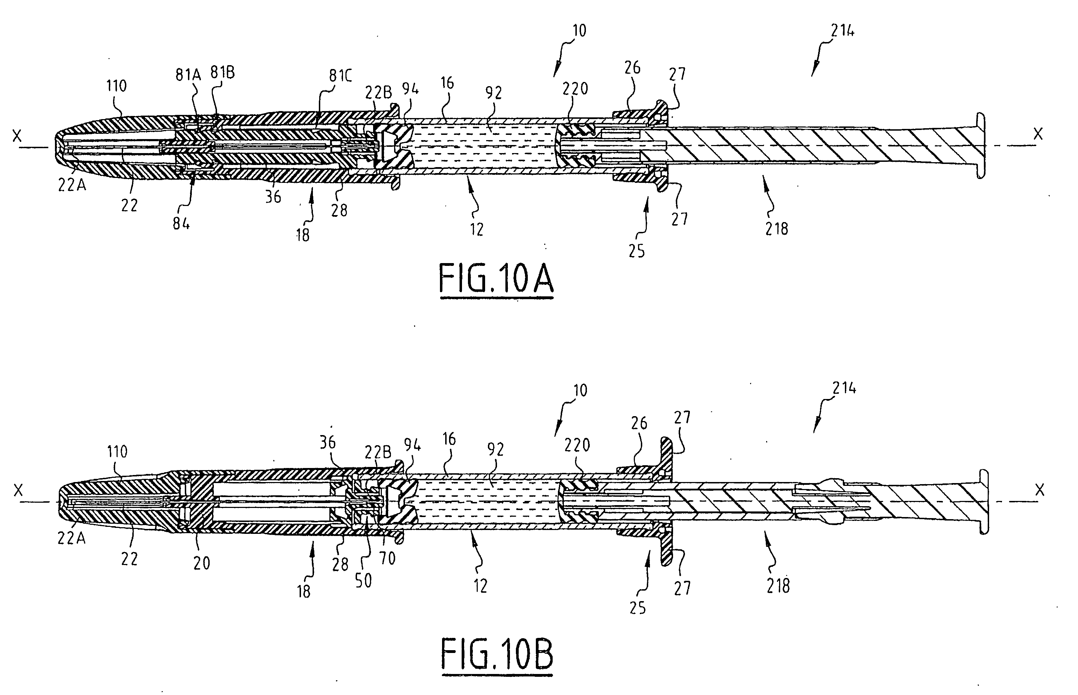

Sur les figures 10 à 13 est illustrée une autre variante de réalisation d'une seringue selon l'invention.

Ce mode de réalisation diffère uniquement, du mode de réalisation précédent, en ce que le poussoir d'actionnement 14 est remplacé par un poussoir d'actionnement télescopique 214.

Le poussoir télescopique 214 comporte une tige de piston 218 à une extrémité avant de laquelle est monté un piston d'extrémité en forme de cuvette 220 analogue au piston arrière 90 du premier mode de réalisation.

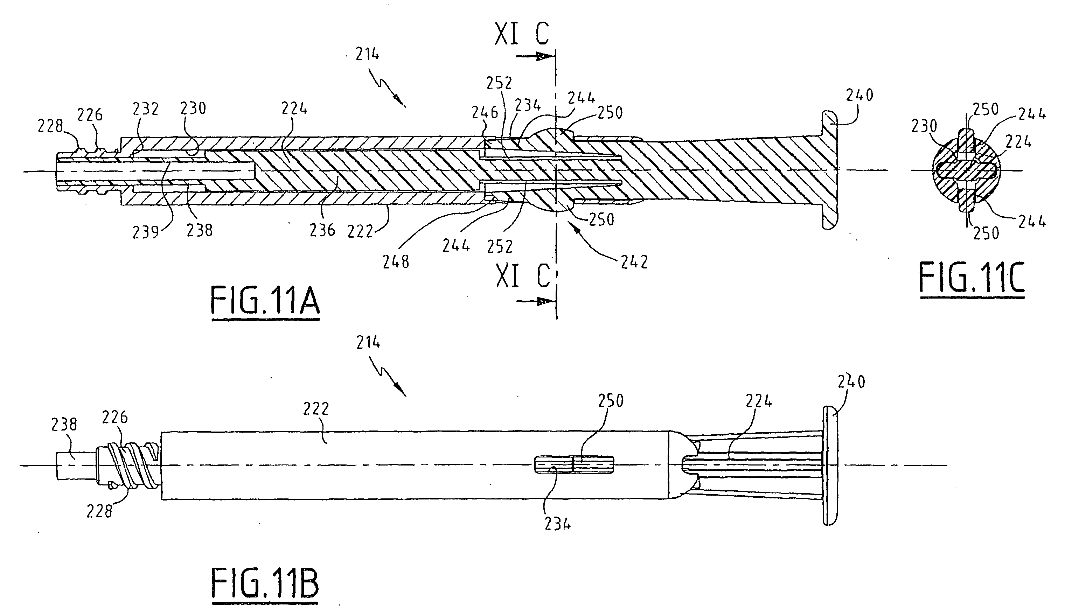

Comme illustré sur les figures 11A, 11B et 11C, la tige de piston 218 comporte un tube extérieur 222 et une tige intérieure 224 montée déplaçable à coulissement à l'intérieur du tube 222.

Le tube extérieur 222 présente un diamètre extérieur très légèrement inférieur au diamètre intérieur du corps de la seringue, c'est-à-dire au diamètre du tube 16 et à la section de passage au travers de l'organe de préhension 25. A son extrémité avant, le tube 222 présente un collet 226 prolongeant axialement le tube. Sur la surface extérieure du collet est ménagé un filetage 228 pour l'accrochage du piston d'extrémité 220.

Le tube 222 délimite intérieurement un conduit 230 à l'intérieur duquel est montée coulissante la tige intérieure 224. Le conduit 230 débouche à ses deux extrémités.

Le conduit 230 présente, en section, généralement la forme d'une croix, comme illustré sur la figure 11C.

A son extrémité avant, le conduit 230 présente un épaulement 232 au-delà duquel s'étend le collet 226.

Au voisinage de son extrémité arrière, le tube 222 présente deux lumières oblongues 234 propres à recevoir des excroissances radiales de la tige 224.

La tige intérieure 224 possède un corps 236 présentant une section généralement en croix complémentaire à celle du conduit 230 pour assurer un guidage axial et une retenue en rotation de la tige 224 dans le conduit 230.

A son extrémité avant, le corps 236 de la tige est prolongé par un tronçon tubulaire 238 engagé au travers du collet 226. La longueur du tronçon tubulaire 238 est supérieure à la longueur du collet 226. Initialement, c'est-à-dire dans la position des figures 11A et 11B, le tronçon tubulaire 238 est totalement escamoté dans le collet 226 et le conduit 230.

Le tronçon tubulaire 238 délimite intérieurement un logement 239 de réception de l'extrémité arrière de l'aiguille. Ce logement se prolonge à l'intérieur du corps 236 de la tige.

A son extrémité arrière, la tige 224 présente une pastille 240 d'appui du pouce de l'utilisateur.

Selon l'invention, des moyens 242 de solidarisation axiale temporaire de la tige 224 par rapport au tube 222 sont prévus dans le poussoir d'actionnement.

Plus précisément, ces moyens de solidarisation temporaire comportent deux jambes élastiques 244 venues de matière avec la tige intérieure 224. Ces jambes sont reliées depuis leur extrémité arrière au corps 236 de là tige. Elles présentent chacune une extrémité libre 246 tournée vers l'avant du poussoir d'actionnement. Les jambes 244 sont formées de sorte que, par élasticité, leurs extrémités libres 246 tendent à s'écarter de l'axe de la tige 224. Ces extrémités libres 246 sont adaptées pour venir en butée axialement sur des épaulements 248 définis dans l'épaisseur du tube 222 en avant des lumières 234 afin d'assurer l'immobilisation axiale de la tige intérieure par rapport au tube.

Sur leur surface extérieure, les jambes 244 présentent des bossages 250 engagés au travers des lumières 234 et faisant saillie par rapport à la surface latérale du tube 222. Ces bossages 250 présentent suivant la direction de l'axe du poussoir une hauteur progressivement croissante de l'avant vers l'arrière formant ainsi une surface de came convexe s'écartant progressivement de l'axe du poussoir de l'avant vers l'arrière.

Des logements 252 sont ménagés dans le corps 236 de la tige intérieure au droit des jambes 244 pour permettre leur escamotage partiel à l'intérieur du tube 222 lorsque celles-ci sont rapprochées de l'axe de la tige intérieure.

La position des lumières 234, ainsi que la position des bossages 250 sur les jambes 244 sont choisies de sorte que, en fin d'injection, alors que le poussoir d'actionnement est en appui contre le piston avant 94, les bossages 250 entrent en contact avec l'extrémité arrière du corps de seringue, et plus particulièrement avec l'extrémité arrière de l'organe de préhension 25.

Pour procéder à l'injection avec une telle seringue, les mêmes étapes que celles décrites en regard des figures 1A, 1B et 4A, 4B sont d'abord mises en oeuvre. Lors de la phase d'injection, les deux éléments coulissants du poussoir télescopique sont maintenus solidaires axialement du fait de l'appui des extrémités libres 246 des jambes sur les épaulements formant butées 248 ménagés dans le tube extérieur 222. Ainsi, la poussée appliquée sur la pastille 240 est transmise au tube 222, lequel repousse le piston arrière 220.

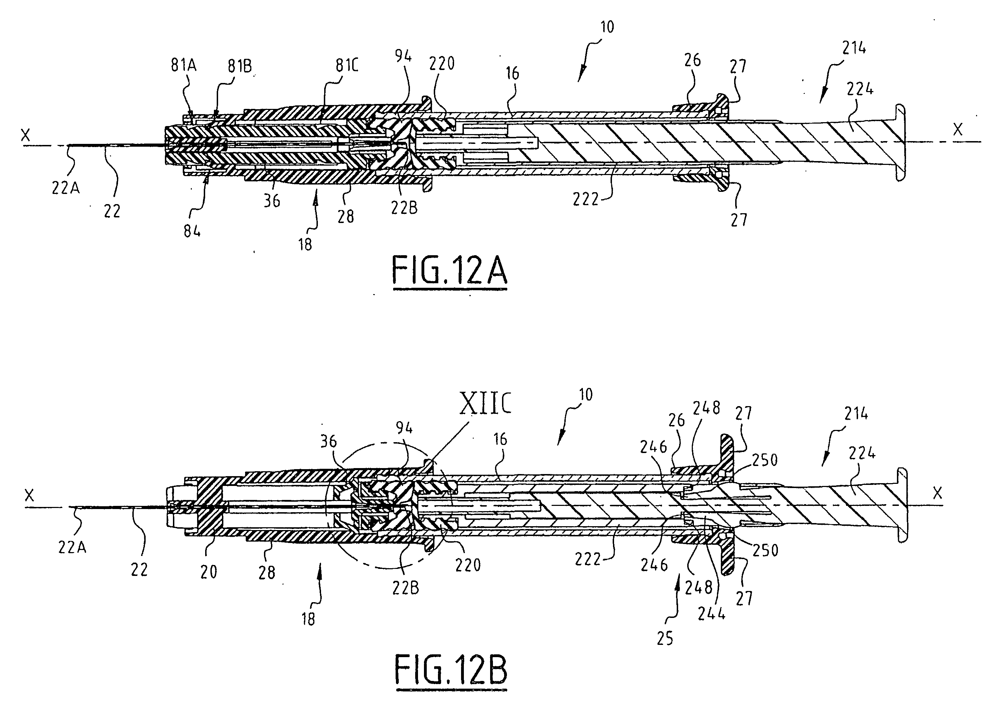

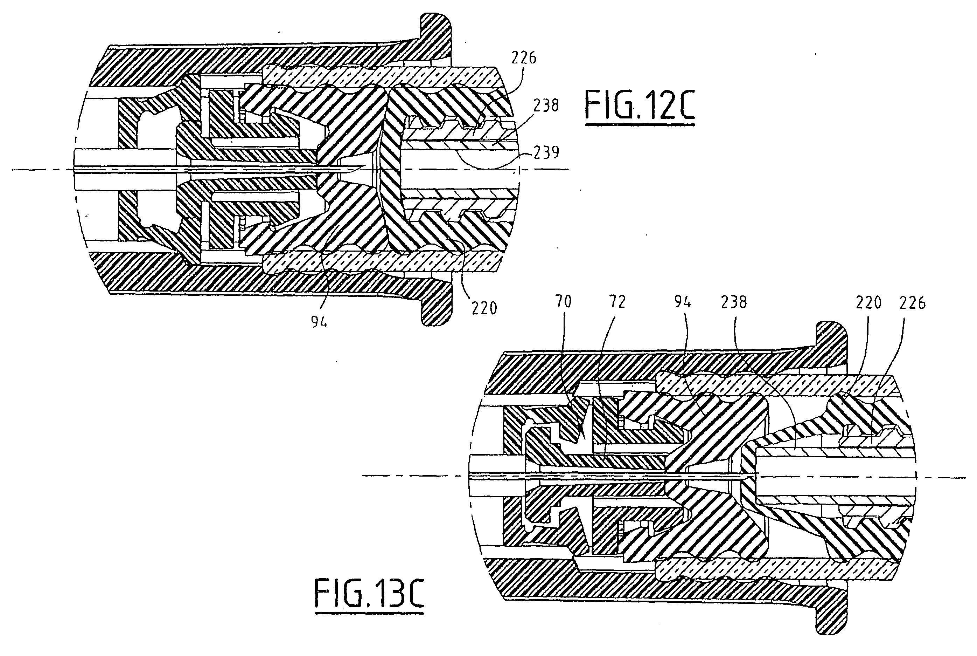

Comme illustré sur les figures 12A et 12B, alors que le piston arrière 220 est en contact avec le piston avant 94, comme illustré sur la figure 12C, les bossages 250 ménagés sur les jambes élastiques 244 de la tige sont repoussés vers l'intérieur du tube 222 par effet de came en entrant en contact avec l'appui-doigt 27. Les jambes 244 sont ainsi déformées, de sorte que leurs extrémités libres 246 se trouvent dégagées des épaulements 248 et sont repoussées dans le logement 252. Les extrémités libres 246 sont alors effacées dans le prolongement du corps 236 de la tige.

Les extrémités 246 des jambes étant écartées des butées 248, le déplacement axial de la tige 224 par rapport au tube 222 est rendu possible, les moyens de solidarisation axiale 242 étant libérés.

Lors de la poursuite de l'appui sur la pastille 240, la poussée n'est plus transmise au tube 220, lequel est maintenu immobile en appui contre le piston avant 94. Au contraire, la tige 224 se déplace axialement à l'intérieur du tube 222. Sous l'effet de ce déplacement, le tronçon tubulaire 238 fait progressivement saillie au-delà du collet 226.

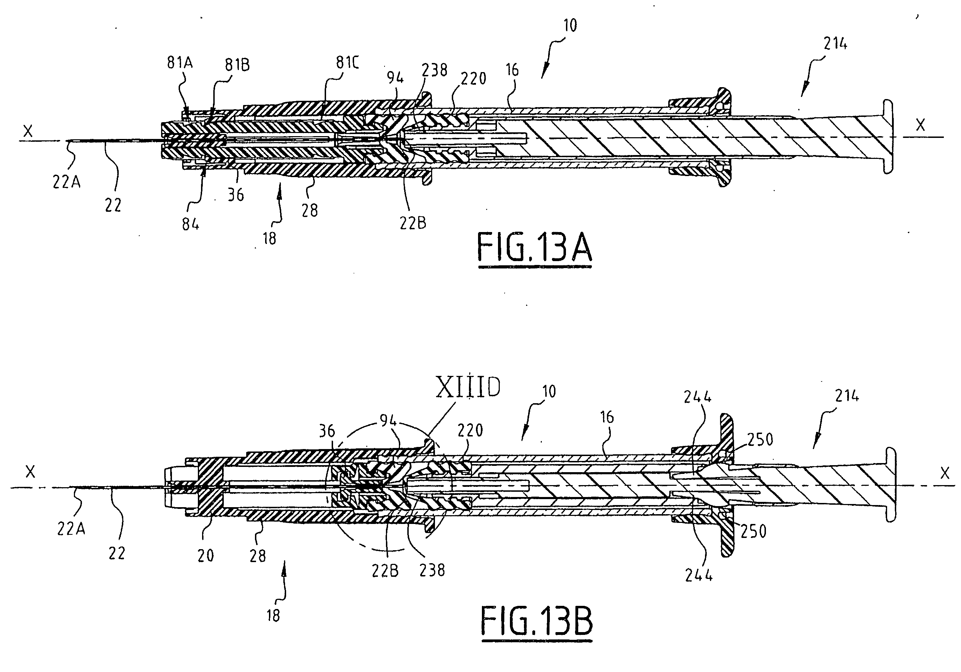

Comme illustré sur les figures 13A, 13B et 13C, l'avancée du tronçon tubulaire 238 provoque une déformation du piston arrière 220. La partie saillante du tronçon tubulaire 238 sollicite axialement l'organe de retenue 70 en appuyant sur son extrémité arrière 74. Comme expliqué en regard de la figure 6C, l'organe de retenue 70 est désolidarisé du protecteur d'aiguille et se déplace axialement vers une position escamotée. Ainsi, les extrémités libres des bras 64 se trouvent libérées, de sorte que le protecteur d'aiguille peut être déplacé vers l'avant.

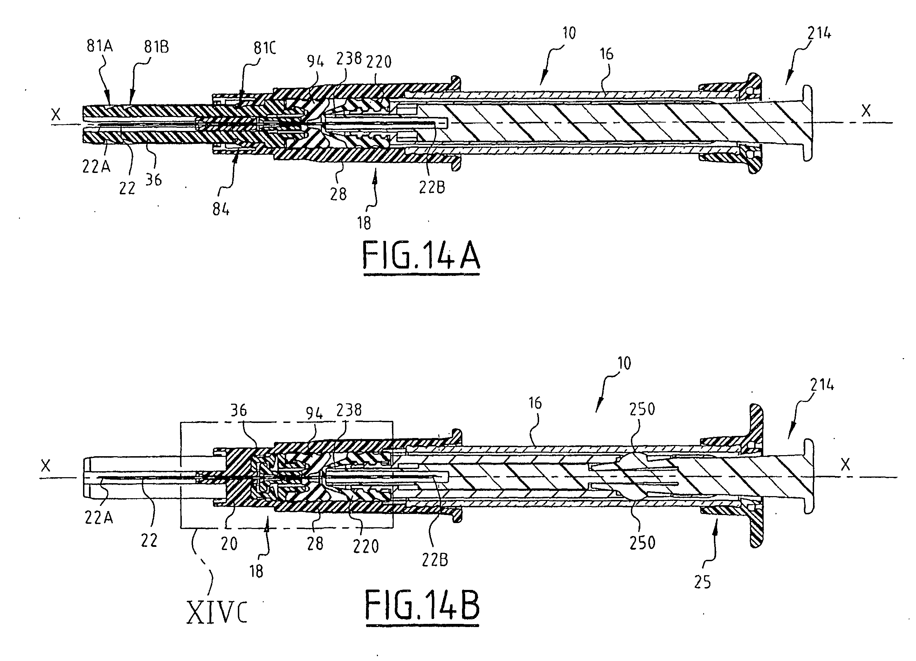

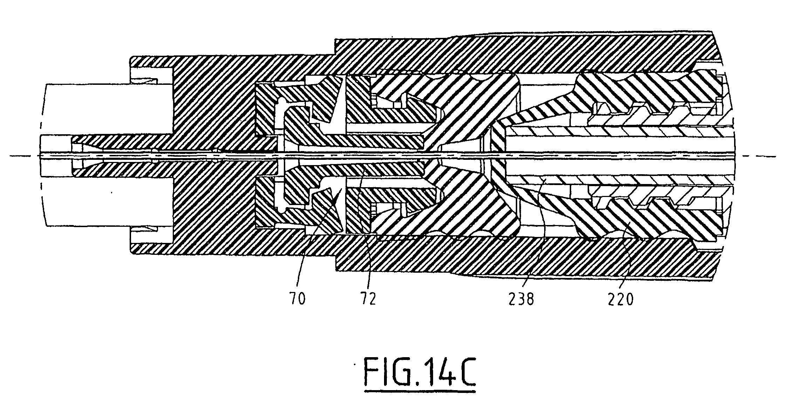

La poursuite de l'appui sur la pastille 240 provoque le déplacement de l'ensemble du poussoir d'actionnement 214 et du protecteur d'aiguille jusqu'à ce que les pistons avant et arrière se trouvent dans la partie avant du corps de la seringue, comme illustré sur les figures 14A, 14B et 14C.

On comprend que l'utilisation d'un poussoir télescopique, dont. les deux parties mobiles sont initialement solidarisées axialement, ces deux parties étant libérées à l'issue de la phase d'injection, permet d'éviter de devoir dimensionner de manière précise la protubérance axiale fixe 88 prévue à l'extrémité du poussoir du premier mode de réalisation. En effet, la longueur de cette protubérance doit être ajustée précisément afin de ne pas être trop longue, ce qui peut occasionner des fuites latérales autour du piston 90 et ne pas être trop courte, ce qui peut rendre difficile ou impossible le déplacement de l'organe de retenue 70.

De plus, l'usage du poussoir télescopique permet de réduire la force nécessaire au déplacement de l'organe de retenue 70. En effet, le poussoir télescopique 214 n'agit que sur la partie centrale du piston 94 par l'intermédiaire du tronçon tubulaire 238 mobile par rapport au tube 222. En revanche, dans le premier mode de réalisation, le poussoir s'appuie sur toute la surface transversale du piston 94. La force nécessaire pour sa déformation est donc importante.

## Revendications

1. Seringue d'injection comportant un corps de seringue (12) muni d'une aiguille d'injection (22) et un poussoir d'actionnement (14 ; 214) monté déplaçable dans le corps (12), la seringue comportant un protecteur mobile (36) de protection de l'extrémité d'injection (22A) de l'aiguille, lequel protecteur (36) est déplaçable par rapport au corps (12) entre une position escamotée dans le corps (12) en retrait de l'extrémité d'injection de l'aiguille et une position active de protection dans laquelle l'extrémité avant du protecteur (36) se trouve en avant de l'extrémité d'injection (22A) de l'aiguille, le protecteur (36) et le corps (12) comportant des reliefs associés (30, 66) en saillie et en creux de maintien du protecteur dans une position escamotée, **caractérisée en ce qu'**elle comporte un organe escamotable (70) de retenue positive de l'engagement desdits reliefs associés (30, 66) en saillie et en creux, lorsque le protecteur (36) est dans une position escamotée, et **en ce que** le poussoir d'actionnement (14) et ledit organe de retenue (70) sont adaptés pour un escamotage dudit organe de retenue (70) par rapport au protecteur d'aiguille (36), sous l'action de l'enfoncement du poussoir d'actionnement (14 ; 214) dans le corps, en fin d'injection, assurant une libération de la retenue positive de l'engagement des reliefs associés (30, 66) en saillie et en creux.

2. Seringue selon la revendication 1, **caractérisée en ce que** le protecteur d'aiguille (36) et l'organe escamotable de retenue (70) sont venus de matière.

3. Seringue selon la revendication 1 ou 2, **caractérisée en ce que** l'organe escamotable de retenue (70) et le protecteur d'aiguille (36) sont initialement solidarisés par un lien frangible (80).

4. Seringue selon l'une quelconque des revendications précédentes, **caractérisée en ce que** lesdits reliefs associés (30, 66) en saillie et en creux comportent au moins un évidement (30) ménagé dans le corps de seringue (12) et au moins un bras élastique (64) portant une saillie extérieure (66) adaptée pour être reçue dans un évidement (30) associé du corps, et **en ce que** ledit organe de retenue (70) comporte au moins une butée (78) de maintien de la ou chaque saillie extérieure (66) dans l'évidement associé (30), avant escamotage dudit organe de retenue (70).

5. Seringue selon la revendication 4, **caractérisée en ce que** lesdits bras élastiques (64) sont ménagés par le tronçon d'extrémité arrière du protecteur d'aiguille tourné vers le piston arrière d'actionnement (14).

6. Seringue selon l'une quelconque des revendications précédentes, **caractérisée en ce que** ladite aiguille d'injection (22) se prolonge axialement à l'intérieur du corps (12) jusqu'à une extrémité arrière (22B) à l'écart de laquelle est initialement disposée, dans le corps, une paroi transversale perforable (94), l'aiguille (22) et ladite paroi transversale (94) étant déplaçables l'une par rapport à l'autre depuis une position initiale dans laquelle la paroi transversale (94) est écartée de l'aiguille (22) jusqu'à une position finale dans laquelle la paroi transversale (94) est transpercée par ladite aiguille (22).

7. Seringue selon la revendication 6, **caractérisée en ce que** ledit organe de retenue (70) comporte un passage (76) de guidage axial de l'extrémité arrière (22B) de l'aiguille, lors de la perforation de la paroi transversale (94).

8. Seringue selon la revendication 6 ou 7, **caractérisée en ce que** ladite paroi transversale (94) est déformable axialement sous l'action du poussoir d'actionnement (14) agissant sur ledit organe de retenue (70).

9. Seringue selon l'une quelconque des revendications 6 à 8, **caractérisée en ce que** ledit protecteur d'aiguille (36) et ladite paroi transversale (94) sont initialement espacés l'un de l'autre, et **en ce qu'**ils comportent des profils complémentaires (58, 101) de solidarisation axiale par enclenchement élastique.

10. Seringue selon l'une quelconque des revendications 6 à 9, **caractérisée en ce que** le protecteur d'aiguille (36) et la paroi transversale (94) comportent des moyens (58, 101 ) de solidarisation axiale au moins lorsque l'aiguille (22) et la paroi transversale sont dans leur position finale, et **en ce que** le protecteur d'aiguille (36) et le corps de seringue (12) comportent des profils complémentaires (81B, 84) en saillie et en creux interdisant le déplacement du protecteur d'aiguille (36) vers le poussoir d'actionnement (14) lorsque l'aiguille (22) et la paroi transversale (94) sont dans leur position finale.

11. Seringue selon l'une quelconque des revendications précédentes, **caractérisée en ce que** le protecteur d'aiguille (36) comporte au moins deux jambes (40) s'étendant généralement parallèlement, lesquelles jambes (40) sont venues de matière et reliées l'une à l'autre à leur extrémité arrière tournée vers le poussoir d'actionnement (14).

12. Seringue selon la revendication 11, **caractérisée en ce que** le protecteur d'aiguille (36) comporte une bague (130) rapportée reliant les extrémités des jambes opposées à leur extrémité tournée vers le piston d'actionnement.

13. Seringue selon l'une quelconque des revendications précédentes, **caractérisée en ce que** le protecteur d'aiguille (36) et le corps de seringue (12) comportent des profils complémentaires (81A, 84) en saillie et en creux interdisant le déplacement du protecteur d'aiguille (36) vers le poussoir d'actionnement (14) lorsque le protecteur d'aiguille est dans sa position escamotée initiale.

14. Seringue selon l'une quelconque des revendications précédentes, **caractérisée en ce que** le corps de seringue (12) comporte au moins une lame élastique (84) et le protecteur d'aiguille (36) comporte au moins une encoche (81C) adaptée pour coopérer avec ladite lame élastique (84) afin d'interdire le déplacement du protecteur d'aiguille (36) vers le poussoir d'actionnement (14) lorsque le protecteur d'aiguille (36) est dans sa position active de protection.

15. Seringue d'injection selon l'une quelconque des revendications précédentes, **caractérisée en ce que** le protecteur d'aiguille (36) est déplaçable par rapport au corps de seringue (12) sous l'action de l'enfoncement progressif du poussoir d'actionnement (14) dans le corps de seringue (12).

16. Seringue d'injection selon l'une quelconque des revendications précédentes, **caractérisée en ce que** le poussoir d'actionnement (214) comporte deux éléments télescopiques (222, 224) montés mobiles axialement l'un par rapport à l'autre et des moyens libérables (242) de solidarisation axiale des deux éléments (222, 224) l'un par rapport à l'autre pendant la phase d'injection, et **en ce qu'**elle comporte des moyens (250) de libération des moyens (246, 248) de solidarisation axiale des deux éléments (222, 224) en fin d'injection.

17. Seringue d'injection selon la revendication 16, **caractérisée en ce que** le poussoir d'actionnement (214) comporte un tube extérieur (222) et une tige intérieure (224) propre à coulisser à l'intérieur du tube extérieur (222), laquelle tige intérieure (224) comporte une extrémité arrière faisant saillie hors du tube extérieur (222) et présentant une surface d'appui (240) d'un doigt pour l'enfoncement du poussoir d'actionnement (224) dans le corps de seringue (12), et **en ce que** la tige intérieure (224) présente un prolongement (238) déplaçable, après libération des moyens de solidarisation axiale (246, 248), entre une position d'injection dans laquelle ledit prolongement (238) est au moins partiellement escamoté dans le tube extérieur (222) et une position active d'escamotage de l'organe de retenue (70) dans laquelle lé prolongement (238) fait saillie par rapport au tube extérieur (222).

18. Seringue d'injection selon la revendication 17, **caractérisée en ce que** les moyens libérables (242) de solidarisation axiale comportent au moins une lumière (234) ménagée dans le tube extérieur (222) et au moins un organe en saillie (244) solidaire de la tige intérieure (224), le ou chaque organe en saillie (244) étant enclenché élastiquement dans une lumière (234), la ou chaque lumière présentant, à une extrémité, une butée (248) d'immobilisation axiale du tube extérieur (222) pour l'appui de la saillie (244) engagée dans la lumière (234).

19. Seringue d'injection selon l'une quelconque des revendications 16 à 18, **caractérisée en ce que** les moyens de libération des moyens (246, 248) de solidarisation axiale des deux éléments (222, 224) en fin d'injection, comportent au moins un organe de déclenchement (250) propre à coopérer avec le corps (12) de seringue en fin d'injection pour provoquer la libération des moyens (246, 248) de solidarisation axiale.

## Patentansprüche

1. Injektions-Spritze mit einem Spritzen-Körper (12), der mit einer Injektions-Kanüle (22) versehen ist und einem Betätigungs-Drücker (14; 214), welcher in dem Körper (12) verschiebbar montiert ist, wobei die Spritze eine bewegbare Schutzvorrichtung (36) zum Schutz des Injektions-Endes (22A) der Kanüle aufweist, welche Schutzvorrichtung (36) bezüglich des Körpers (12) zwischen einer von dem Injektions-Ende der Kanüle in den Körper zurückgezogenen Einfahr-Position und einer aktiven Schutzposition verschiebbar ist, in welcher sich das Vorder-Ende der Schutzvorrichtung (36) vor dem Injektions-Ende (22A) der Kanüle befindet, wobei die Schutzvorrichtung (36) und der Körper (12) assoziierte Schulterflächen und Vertiefungsflächen (30, 66) aufweisen zum Festhalten der Schutzvorrichtung in einer Einfahr-Position, **dadurch gekennzeichnet, dass** sie ein versenkbares Bauteil (70) zum sicheren Halten des Eingriffs der assoziierten Schulterflächen und Vertiefungsflächen (30, 66) aufweist, wenn die Schutzvorrichtung (36) in einer Einfahr-Position ist, und dass der Betätigungs-Drücker (14) und das Halte-Bauteil (70) eingerichtet sind zum Versenken des Halte-Bauteils (70) bezüglich der Kanülen-Schutzvorrichtung (36) unter der Einwirkung der Eintreibung des Betätigungs-Drückers (14; 214) in den Körper hinein, was am Ende der Injektion eine Freigabe des sicheren Halts des Eingriffs der assoziierten Schulterflächen und Vertiefungsflächen (30, 66) gewährleistet.

2. Spritze gemäß Anspruch 1, **dadurch gekennzeichnet, dass** die Kanülen-Schutzvorrichtung (36) und das versenkbare Halte-Bauteil (70) aus einem Stück sind.

3. Spritze gemäß Anspruch 1 oder 2, **dadurch gekennzeichnet, dass** das versenkbare Halte-Bauteil (70) und die Kanülen-Schutzvorrichtung (36) anfänglich mittels eines brechbaren Verbindungsteils (80) verbunden sind.

4. Spritze gemäß einem der vorhergehenden Ansprüche, **dadurch gekennzeichnet, dass** die assoziierten Schulterflächen und Vertiefungsflächen (30, 66) mindesten eine Aussparung (30), welche in dem Spritzen-Körper (12) angeordnet ist, und mindestens einen elastischen Arm (64) aufweisen, welcher eine außenliegende Schulter (66) trägt, welcher zum Unterbringen in einer im Körper vorgesehenen Ausnehmung (30) unterbringbar ist, und dass das Halte-Bauteil (70) mindestens einen Anschlag (78) aufweist zum Halten der oder jeder außenliegenden Schulter (66) in der assoziierten Ausnehmung (30) vor der Versenkung das Halte-Bauteils (70).

5. Spritze gemäß Anspruch 4, **dadurch gekennzeichnet, dass** die elastischen Arme (64) von dem hinteren Endabschnitt der Kanülen-Schutzvorrichtung gebildet sind, welcher hin zu dem hinteren Antriebs-Kolben (14) gewandt ist.

6. Spritze gemäß einem der vorhergehenden Ansprüche, **dadurch gekennzeichnet, dass** die Injektions-Kanüle (22) sich axial im Inneren des Körpers (12) bis zu einem hinteren Ende (22B) erstreckt, im Abstand zu welchem anfänglich im Körper eine durchstechbare Querwand (94) angeordnet ist, wobei die Kanüle (22) und die Querwand (94) relativ zueinander von einer Anfangsposition, in welcher die Querwand (94) zu der Kanüle (22) mit einem Abstand angeordnet ist, bis zu einer Endposition verschiebbar sind, in welcher die Querwand (94) von der Kanüle (22) durchstochen ist.

7. Spritze gemäß Anspruch 6, **dadurch gekennzeichnet, dass** das Halte-Bauteil (70) eine Passage (76) zum axialen Führen des hinteren Endes (22B) der Kanüle beim Durchstechen der Querwand (94) aufweist.

8. Spritze gemäß dem Anspruch 6 oder 7, **dadurch gekennzeichnet, dass** die Querwand (94) unter der Einwirkung des Betätigungs-Drückers (14) bewirkt durch das Halte-Bauteil (70) axial deformierbar ist.

9. Spritze gemäß einem der Ansprüche 6 bis 8, **dadurch gekennzeichnet, dass** die Kanülen-Schutzvorrichtung (36) und die Querwand (94) anfänglich mit einem Zwischenraum zueinander angeordnet sind, und dass sie komplementäre Konturen (58, 101) zum axialen Verbinden mittels elastischer Verriegelung aufweisen.

10. Spritze gemäß einem der Ansprüche 6 bis 9, **dadurch gekennzeichnet, dass** die Kanülen-Schutzvorrichtung (36) und die Querwand (94) Mittel (58, 101) zum axialen Verbinden aufweisen, zumindest wenn die Kanüle (22) und die Querwand in deren Endposition sind, und dass die Kanülen-Schutzvorrichtung (36) und der Spritzen-Körper (12) komplementäre Schulterkonturen und Vertiefungskonturen (81B, 84) aufweisen, welche die Verschiebung der Kanülen-Schutzvorrichtung (36) hin zu dem Betätigungs-Drücker (14) sperren, wenn die Kanüle (22) und die Querwand (94) in deren Endposition sind.

11. Spritze gemäß einem der vorhergehenden Ansprüche, **dadurch gekennzeichnet, dass** die Kanülen-Schutzvorrichtung (36) mindestens drei Schenkel (40) aufweist, die sich im Wesentlichen parallel erstrecken, welche Schenkel (40) aus einem Stück bestehen und an deren hinteren Enden miteinander verbunden sind, welche hin zu dem Betätigungs-Drücker (14) geneigt sind.

12. Spritze gemäß Anspruch 11, **dadurch gekennzeichnet, dass** die Kanülen-Schutzvorrichtung (36) einen aufgesteckten Ring (130) aufweist, welcher jene Enden der Schenkel verbindet, die deren dem Betätigungs-Kolben zugewandten Ende entgegengesetzt sind.

13. Spritze gemäß einem der vorhergehenden Ansprüche, **dadurch gekennzeichnet, dass** die Kanülen-Schutzvorrichtung (36) und der Spritzen-Körper (12) komplementäre Schulterkonturen und Vertiefungskonturen (81A,84) aufweisen, welche die Verschiebung der Kanülen-Schutzvorrichtung (36) hin zu dem Betätigungs-Drücker (14) sperren, wenn die Kanülen-Schutzvorrichtung in ihrer anfänglichen Einfahr-Position ist.

14. Spritze gemäß einem der vorhergehenden Ansprüche, **dadurch gekennzeichnet, dass** der Spritzenkörper (12) mindestens eine elastische Zunge (84) aufweist und die Kanülen-Schutzvorrichtung (36) mindestens eine Raste (81C) aufweist, welche zum Zusammenwirken mit der elastischen Zunge (84) eingerichtet ist, um die Verschiebung der Kanülen-Schutzvorrichtung (36) hin zu dem Betätigungs-Drücker (14) zu sperren, wenn die Kanülen-Schutzvorrichtung (36) in ihrer aktiven Schutzposition ist.

15. Injektions-Spritze gemäß einem der vorhergehenden Ansprüche, **dadurch gekennzeichnet, dass** die Kanülen-Schutzvorrichtung (36) bezüglich des Spritzen-Körpers (12) unter der Einwirkung des progressiven Eintreibens des Betätigungs-Drückers (14) in den Spritzenkörper (12) hinein verschiebbar ist.

16. Injektions-Spritze gemäß einem der vorhergehenden Ansprüche, **dadurch gekennzeichnet, dass** der Betätigungs-Drücker (214) zwei teleskopische Elemente (222,224), die axial bewegbar zueinander montiert sind, und freigebbare Mittel (242) aufweist zum axialen Verbinden der beiden Elemente (222,224) aneinander während der Injektions-Phase, und dass sie Mittel (250) aufweist zum Freigeben der Mittel (246,248) zum axialen Verbinden der beiden Elemente (222,224) am Ende der Injektion.

17. Injektions-Spritze gemäß Anspruch 16, **dadurch gekennzeichnet, dass** der Betätigungs-Drücker (214) eine Außenhülse (222) und einen Innenstift (224) aufweist, der zum Gleiten im Inneren der Außenhülse (222) geeignet ist, welcher Innenstift (224) ein hinteres Ende aufweist, welches außerhalb der Außenhülse (222) hervorsteht und eine DaumenAuflagefläche (240) aufweist zum Eintreiben des Betätigungs-Drückers (224) in den Spritzenkörper (12) hinein, und dass der Innenstift (224) einen Fortsatz (238) aufweist, welcher nach der Freigabe der Mittel (246,248) zum axialen Verbinden zwischen einer Injektions-Position, in welcher der Fortsatz (238) zumindest teilweise in der Außenhülse (222) versenkt ist, und einer Aktiv-Position zum Versenken des Halte-Bauteils (70) verschiebbar ist, in welcher der Fortsatz (238) bezüglich der Außenhülse (222) hervorsteht.

18. Injektions-Spritze gemäß Anspruch 17, **dadurch gekennzeichnet, dass** die freigebbaren Mittel (242) zum axialen Verbinden mindestens ein Langloch (234), welches in der Außenhülse (222) vorgesehen ist, und mindestens ein Schulter-Stück (244) aufweisen, welches einstückig mit dem Innenstift (224) ist, wobei das oder jedes Schulter-Stück (244) in einem Langloch (234) elastisch eingerastet ist, wobei das oder jedes Langloch an einem Ende einen Anschlag (248) zum axialen Immobilisieren der Außenhülse (222) aufweist zum Halten des in dem Langloch (234) eingesetzten Ansatzes (244).

19. Injektions-Spritze gemäß einem der Ansprüche 16 bis 18, **dadurch gekennzeichnet, dass** die Mittel zum Freigeben der Mittel (246,248) zum axialen Verbinden der beiden Elemente (222,224) am Ende der Injektion mindestens ein Auskuppel-Stück (250) aufweisen, welches geeignet ist, mit dem Körper (12) der Spritze am Ende der Injektion zusammenzuwirken zum Provozieren der Freigabe der Mittel (246,248) der axialen Verbindung.

## Claims

1. Injection syringe comprising a syringe body (12) provided with an injection needle (22) and an actuating plunger (14; 214) displaceably mounted in the body (12), the syringe including a moveable protector (36) for protecting the injection end (22A) of the needle, which protector (36) can be displaced.with respect to the body (12) between a retracted position in the body (12) set back from the injection end of the needle and an active protection position in which the front end of the protector (36) is situated in front of the injection end (22A) of the needle, the protector (36) and the body (12) including associated projecting and hollowed-out portions in relief (30, 66) for holding the protector in the retracted position, **characterized in that** it includes a retractable device (70) for positively retaining the engagement of the said associated projecting and hollowed out portions in relief (30, 66) when the protector (36) is in the retracted position, and **in that** the actuating plunger (14) and the said retaining device (70) are adapted so as to retract the said retaining device (70) with respect to the needle protector (36) under the action of forcing the actuating plunger (14; 214) into the body at the end of injection, ensuring release of the associated projecting and hollowed out portions in relief (30, 66) from being positively retained in engagement.

2. Syringe according to claim 1, **characterized in that** the needle protector (36) and the retaining retractable device (70) are made in one piece.

3. Syringe according to claim 1 or 2, **characterized in that** the retaining retractable device (70) and the needle protector (36) are initially interlocked by a breakable linkage (80)

4. Syringe according to any one of the preceding claims, **characterized in that** the said associated projecting and hollowed-out portions in relief (30, 66) include at least one recess (30) provided in the body of the syringe (12) and at least one elastic arm (64) carrying an outer projection (66) adapted so as to be received in an associated recess (30) of the body, and **in that** the said retaining device (70) includes at least one stop (78) for holding the or each outer projection (66) in the associated recess (30), before the said retaining device (70) is retracted.

5. Syringe according to claim 4, **characterized in that** the said elastic arms (64) are provided by the rear end section of the needle protector turned towards the rear actuating piston (14).

6. Syringe according to any one of the preceding claims, **characterized in that** the said injection needle (22) is extended axially inside the body (12) as far as a rear end (22B) separated from which a perforable transverse wall (94) is initially positioned in the body, the needle (22) and the said transverse wall (94) being displaceable in relation to each other from an initial position in which the transverse wall (94) is separated from the needle (22) up to a final position in which the transverse wall (94) is pierced through by the said needle (22).

7. Syringe according to claim 6, **characterized in that** the said retaining device (70) includes a passage (76) for axially guiding the rear end (22B) of the needle as the transverse wall is perforated (94).

8. Syringe according to claim 6 or 7, **characterized in that** the said transverse wall (94) can be deformed axially by the action of the actuating plunger (14) acting on the said retaining device (70).

9. Syringe according to any one of claims 6 to 8, **characterized in that** the said needle protector (36) and the said transverse wall (94) are initially spaced from each other, and **in that** they include complementary profiles (58, 101) for axial interlocking by elastic engagement.

10. Syringe according to any one of claim 6 to 9, **characterized in that** the needle protector (36) and the transverse wall (94) include axial means (58, 101) for axial interlocking, at least when the needle (22) and the transverse wall are in their final position, and **in that** the needle protector (36) and the syringe body (12) include projecting and hollowed-out complementary profiles (81B, 84) for preventing the displacement of the needle protector (36) towards the actuating plunger (14) when the needle (22) and the transverse wall (94) are in their final position.

11. Syringe according to any one of the preceding claims, **characterized in that** the needle protector (36) includes at least two legs (40) extending in a generally parallel manner, which legs (40) are made in one piece and are connected to each other at their rear end turned towards the actuating plunger (14).

12. Syringe according to claim 11, **characterized in that** the needle protector (36) includes an added ring (130) linking the ends of the legs opposite their end turned towards the actuating piston.

13. Syringe according to any one of the preceding claims, **characterized in that** the needle protector (36) and the syringe body (12) include complementary projecting and hollowed-out profiles (81A, 84) preventing displacement of the needle protector (36) towards the actuating plunger (14) when the needle protector is in its initial retracted position.

14. Syringe according to any one of the preceding claims, **characterized in that** the syringe body (12) includes at least one elastic blade (84) and the needle protector (36) includes at least one notch (81C) adapted so as to cooperate with the said elastic blade (84) in order to prevent displacement of the needle protector (36) towards the actuating plunger (14) when the needle protector (36) is in its active protecting position.

15. Injection syringe according to any one of the preceding claims, **characterized in that** the needle protector (36) is displaceable with respect to the syringe body (12) under the action of progressively forcing the actuating plunger (14) into the body of the syringe (12).

16. Syringe according to any one of the preceding claims, **characterized in that** the actuating plunger (214) includes telescopic elements (222, 224) mounted so as to move axially with respect to each other and releasable means (242) for axially interlocking the two elements (222, 224) with respect to each other during the injection phase, and **in that** it includes means (250) for releasing the means (246, 248) for axially interlocking the two elements (222, 224) at the end of injection.

17. Injection syringe according to claim 16, **characterised in that** the actuating plunger (214) includes an outer tube (222) and an inner rod (224) capable of sliding inside the outer tube (222), which inner rod (224) has a rear end projecting outside the outer tube (222) and having a surface (240) for supporting a finger for forcing the actuating plunger (224) into the body of the syringe (12), and **in that** the inner rod (224) has an extension (238) that can be displaced, after releasing the axial interlocking means (246, 248), between an injection position in which the said extension (238) is at least partially retracted inside the outer tube (222) and an active retracted position of the retaining device (70) in which the extension (238) projects with respect to the outer tube (222).

18. Injection syringe according to claim 17, **characterised in that** the releasable axial interlocking means (242) include at least one opening (234) provided in the outer tube (222) and at least one projecting component (244) secured to the inner rod (224), the or each projecting component (224) being elastically engaged in an opening (234), the or each opening having, at one end, a stop (248) for axially immobilising the outer tube (222) for support of the projection (244) engaged in the opening (234).

19. Injection syringe according to any one of claims 16 to 18, **characterised in that** the means for releasing the means (246, 248) for axially interlocking the two elements (222, 224) at the end of injection, include at least one disengaging device (250) capable of cooperating with the body (12) of the syringe at the end of the injection so as to bring about release of the axial interlocking means (246, 248).
